# DEMANDE DE BREVET EUROPEEN

(11) **EP 3 926 323 A1**
(43) Date de publication de la demande: **22.12.2021**
(21) Numéro de dépôt: 21179095.1
(22) Date de dépôt: 11.06.2021
(51) Int. Cl.: G01N 1/20, G01N 33/18, G01N 1/12, G01N 1/14

(54) **DISPOSITIF DE PRÉLÈVEMENT EN MILIEU AQUEUX**

(30) Priorité: 15.06.2020 FR 2006216
(71) Demandeur: AD Scientifique, 67410 Rohrwiller (FR)
(72) Inventeur: VICO, Sandra, 67410 ROHRWILLER (FR)
(74) Mandataire: Cabinet Nuss

(57) **Abrégé**

L'invention se rapporte à un dispositif de prélèvement (1) en milieu aqueux, caractérisé en ce que le dispositif (1) comprend:
- une structure support (2) rectiligne,
- une interface d'accumulation montée sur au moins une partie de la structure support (2),
- un moyen de flottaison (4) monté à une extrémité de la structure support (2) rectiligne.

## Description

La présente invention se rapporte au domaine des dispositifs de prélèvement biologique et/ou chimique en milieu aqueux extérieur et plus particulièrement au domaine des dispositifs de prélèvement biologique ou chimique en milieu aqueux en écoulement.

Le processus de prélèvement de matériaux chimiques et biologiques et notamment de biomasse en milieu aqueux est généralement opéré par des prélèvements successifs effectués à intervalles réguliers de façon permettre ultérieurement la mesure de l'évolution des matériaux biologiques et/ou chimiques prélevés. Toutefois, lorsque le milieu étudié se rapporte à un écoulement aqueux ou à des eaux-vives, les prélèvements ponctuels de matériaux biologiques et/ou chimiques montrent leurs limites en ne permettant pas une détection efficace d'éléments présents en trop faible quantité. De façon similaire, de tels prélèvements ponctuels présentent un défaut d'efficacité pour identifier l'apparition d'une éventuelle modification dans la composition du milieu aqueux prélevé et son retour à la normale lorsque ce phénomène est succinct, voire même localisé, et survient au cours de l'intervalle qui sépare deux prélèvements successifs.

La présente invention a pour but de pallier cet inconvénient en proposant une solution qui permette la réalisation de prélèvements dans des milieux aqueux en écoulement susceptible de permettre une identification de matériaux présents à de faibles concentration tout en étant en mesure de répondre aux principales contraintes rencontrées dans le cadre de prélèvements en eaux-vives.

L'invention concerne un dispositif de prélèvement en milieu aqueux, caractérisé en ce que le dispositif comprend :
- une structure support rectiligne,
- une interface d'accumulation montée sur au moins une partie de la structure support,
- un moyen de flottaison monté à une extrémité du support rectiligne.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :
[Fig.1] est une représentation schématique d'un premier exemple de réalisation d'un dispositif de prélèvement en milieu aqueux selon l'invention.
[Fig.2] est une représentation schématique du premier exemple de dispositif de prélèvement en milieu aqueux selon l'invention dans laquelle certains éléments du dispositif sont séparés.
[Fig.3] est une représentation schématique en section du premier exemple d'interface d'accumulation associé à un élément de structure support pour un dispositif de prélèvement selon l'invention.

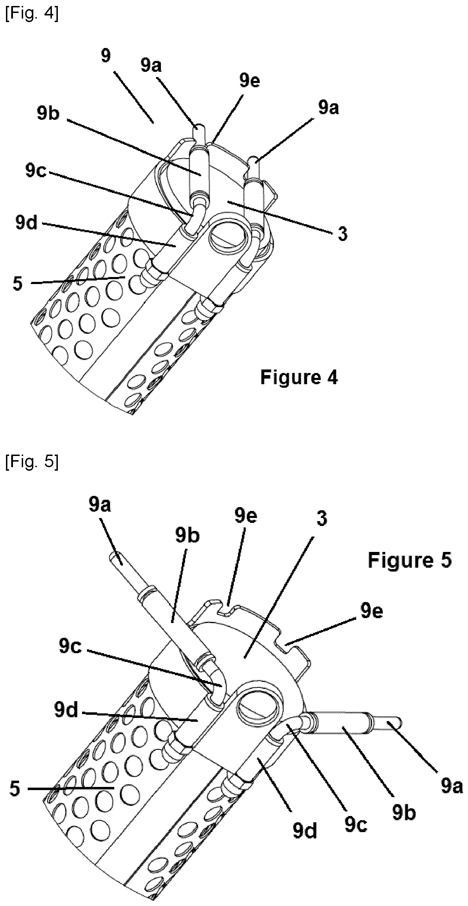

[Fig.4] est une représentation schématique d'un exemple de construction d'une interface de pincement pour un dispositif de prélèvement selon l'invention selon le premier exemple, lorsque l'interface est verrouillée.
[Fig.5] est une représentation schématique d'un exemple de construction d'une interface de pincement pour un dispositif de prélèvement selon l'invention selon le premier exemple, lorsque l'interface est déverrouillée.
[Fig.6] est une représentation schématique en section d'un second exemple de réalisation d'un dispositif de prélèvement en milieu aqueux selon l'invention.
[Fig.7] est une représentation schématique du second exemple de dispositif de prélèvement en milieu aqueux selon l'invention dans laquelle l'interface de pincement est en position avant essorage de l'interface d'accumulation.
[Fig.8] est une représentation schématique du second exemple de dispositif de prélèvement en milieu aqueux selon l'invention dans laquelle l'interface de pincement a été déplacée le long de la structure support pour l'essorage de l'interface d'accumulation.
[Fig.9] est une représentation schématique selon une vue cavalière d'une première face d'une moitié de cône pour la réalisation d'un arrangement conique selon l'invention.
[Fig.10] est une représentation schématique selon une vue cavalière d'une seconde face d'une moitié de cône pour la réalisation d'un arrangement conique selon l'invention.
[Fig.11] est une représentation schématique d'une moitié de cône selon une vue en section dans un plan qui comprend l'axe de translation de l'arrangement conique qui forme l'interface de pincement.
[Fig.12] est une représentation schématique d'une moitié de cône selon une vue en section dans un plan perpendiculaire à l'axe de translation de l'arrangement conique qui forme l'interface de pincement.

L'invention se rapporte à un dispositif de prélèvement 1 en milieu aqueux, caractérisé en ce que le dispositif 1 comprend :
- une structure support 2 rectiligne,
- une interface d'accumulation 3 montée sur au moins une partie de la structure support 2,
- un moyen de flottaison 4 monté à une extrémité de la structure support 2 rectiligne.

Le dispositif de prélèvement 1 selon l'invention est réalisé de façon à pouvoir être traversé, au niveau d'au moins une partie, par le flux aqueux du milieu dans lequel le dispositif 1 est positionné. L'intégration d'une interface d'accumulation 3 dans le dispositif de prélèvement 1 de l'invention permet d'opérer une rétention et la conservation de matériaux susceptibles de n'être présents qu'en faible quantité dans le milieu aqueux où le dispositif de prélèvement 1 se trouve placé. Selon un exemple de construction, l'interface d'accumulation 3 est réalisée selon une forme sensiblement rectiligne.

La construction du dispositif 1 autour d'une structure support 2 de forme rectiligne couplée avec un moyen de flottaison 4 monté, directement ou indirectement, à une extrémité de la structure support 2 permet d'assurer le positionnement du dispositif 1 dans le milieu aqueux à prélever de sorte que, d'une part, une extrémité du dispositif 1 soit maintenue à proximité ou au niveau de la surface aqueuse et que, d'autre part, par gravité, le corps du dispositif de prélèvement 1 de l'invention soit plongé dans le milieu aqueux selon une orientation présentant au moins une composante verticale. Le placement d'un moyen de flottaison 4 en extrémité de la structure support 2 assure un positionnement du dispositif 1 et son maintien de sorte que celui-ci présente une composante verticale. Le moyen de flottaison 4 permet également d'éviter au dispositif de prélèvement 1 de l'invention de couler au fond du milieu dans lequel il est placé, de sorte qu'en reposant à ce niveau, la qualité des prélèvements serait altérée et ceux-ci ne seraient pas représentatifs des matériaux ou éléments en suspension dans le milieu liquide en écoulement. Par, ailleurs, en assurant une orientation du dispositif de prélèvement 1 selon au moins une composante verticale, le moyen de flottaison 4 permet au dispositif 1 d'être positionné en travers du flux ou de l'écoulement dans le milieu aqueux dans lequel il est placé. Aussi, le positionnement du dispositif de prélèvement 1 en travers de la hauteur du flux aqueux permet d'augmenter la surface de l'interface d'accumulation 3 impactée par le milieu liquide en écoulement et d'opérer un prélèvement plus efficace de matériaux en suspension dans l'écoulement du liquide.

Selon un exemple particulier de construction du dispositif de prélèvement 1 de l'invention, et de façon complémentaire, le dispositif 1 comprend également un moyen de lestage 6 monté, directement ou indirectement, au support rectiligne. Préférentiellement, ce moyen de lestage 6 est monté à une extrémité du support rectiligne positionnée à l'opposé du moyen de flottaison 4. Un tel arrangement permet une optimisation du positionnement du dispositif de prélèvement 1 en travers d'un flux, notamment lorsque le débit de celui-ci est particulièrement important et serait susceptible de déplacer le dispositif de prélèvement de l'invention pour l'aligner sur l'axe du flux réduisant en conséquence la surface de l'interface d'accumulation 3 impactée par le débit de liquide.

Selon un autre exemple particulier de construction du dispositif de prélèvement 1 de l'invention, également complémentaire, le dispositif 1 comprend également un moyen de fixation 7 qui permet d'arrimer le dispositif 1 à un point fixe, de façon à empêcher le dispositif 1 d'être emporté par le débit de l'eau en écoulement. Ce moyen de fixation 7 est susceptible de prendre la forme d'un câble d'accroché pour autoriser une certaine liberté de mouvements au dispositif de prélèvement 1 placé dans le courant. Cette liberté de mouvements autorise ainsi au dispositif 1 d'adapter sa position pour s'écarter d'éventuels éléments charriés par le courant, tels que par exemple des branchages, des feuilles ou des déchets.

Selon un autre exemple particulier de construction du dispositif de prélèvement 1 de l'invention, complémentaire des particularités précédemment détaillées, l'interface d'accumulation 3 comprend au moins un matériau de type spongieux. Une telle interface 3 spongieuse, notamment grâce à son arrangement structurel alvéolaire, permet, d'une part, de réaliser un moyen d'absorption et de retenu de matériaux en suspension dans le flux aqueux et, d'autre part, de fournir une surface support sur laquelle un film de matière intégrant des composés biologiques et/ou chimiques est en mesure de se former et de se développer.

Selon un exemple particulier de construction du dispositif de prélèvement 1 de l'invention notamment illustré sur les différentes figures annexées et complémentaire des particularités précédemment détaillées, la structure support 2 est réalisée de façon à présenter au moins une première portion 2a de forme sensiblement axiale et autour de laquelle au moins une partie de l'interface d'accumulation 3 est montée ou fixée. Un tel arrangement permet à l'interface d'accumulation 3 d'assurer un prélèvement et une rétention de matériaux en suspension au niveau de chacune des faces du dispositif 1. Aussi, le dispositif de prélèvement 1 de l'invention est en mesure d'opérer un prélèvement quelle que soit sa position par rapport au flux aqueux au travers duquel est placé le dispositif 1. Selon une spécificité de cet exemple particulier de construction, l'interface d'accumulation 3 présente une longueur supérieure à celle de la portion 2a de forme sensiblement axiale de la structure support 2. Aussi, l'interface d'accumulation 3 n'est guidée par la structure support 2 que sur une portion de sa longueur.

Selon un autre exemple particulier de construction du dispositif de prélèvement 1 de l'invention, complémentaire des particularités précédemment détaillées, la structure support 2 comprend également un moyen de protection 5 qui entoure au moins une partie de l'interface d'accumulation 3. Ce moyen de protection 5 réalise ainsi une coque comprenant une ou plusieurs ouvertures.

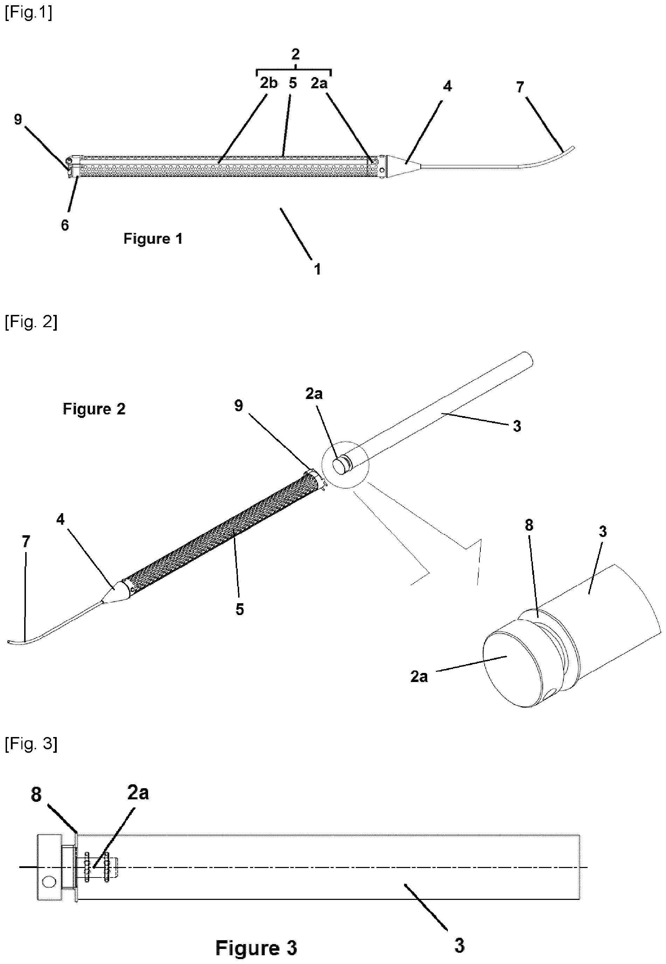

est réalisé sous la forme d'une surface percée de plusieurs orifices ou alternativement d'un grillage ou d'un filtre mécanique, voire même d'une combinaison de ces exemples de surface. Un tel moyen de protection 5 est ainsi arrangé pour autoriser le passage du flux aqueux au travers du dispositif 1 tout en évitant que l'interface d'accumulation 3 soit obturée ou abîmée par des éléments charriés par le courant, tels que par exemple des branchages, des feuilles ou des déchets. Dans le cadre d'un exemple de mise en oeuvre de l'invention, le moyen de protection 5 présente la forme d'une structure creuse rectiligne à l'intérieure de laquelle se trouve positionnée l'interface d'accumulation 3. De façon préférée, afin de faciliter son utilisation, le moyen de protection 5 présente une structure dont la section est de forme cylindrique. Une section de forme cylindrique présente cet avantage d'autoriser un écoulement du flux aqueux autour du dispositif 1 en répondant aux contraintes de dynamique des fluides quel que soit son positionnement. Toutefois, d'autres formes de section sont susceptibles d'être envisagées telles que, à titre d'exemple, rectangulaire, carrée ou triangulaire. Ces différentes formes comprenant des faces sensiblement planes sont susceptibles de faire supporter des contraintes plus importantes au dispositif 1 par rapport à des surfaces arrondies. Par ailleurs, il convient de relever que le moyen de protection 5 présente une section de forme sensiblement complémentaire à la section de la structure support 2 et de l'interface d'accumulation 3. Ainsi, le moyen de protection 5 participe également à la fonction de support de l'interface d'accumulation 3 sous la forme d'une structure de type exosquelette. Selon une spécificité de cette particularité de réalisation, la première portion 2a de forme sensiblement axiale de la structure support 2 et le moyen de protection 5 sont arrangés pour être assemblés entre eux de façon réversible. Selon un exemple de cette spécificité de réalisation, la première portion 2a de forme sensiblement axiale est monté coulissante à l'intérieur du moyen de protection 5, notamment au niveau d'un rail 2b positionné dans la longueur du moyen de protection 5. De façon préférentielle, l'interface d'accumulation 3 est montée sur la première portion 2a de forme sensiblement axiale de sorte que la séparation des deux éléments qui réalise ensemble la structure support 2 entraine également la séparation de l'interface d'accumulation 3 d'avec le moyen de protection 5. Selon un exemple de construction illustré en partie aux figures 1 et 2, le rail 2b est réalisé sous la forme d'une rainure disposée dans l'épaisseur du moyen de protection 5 et dans la longueur de celui-ci. La première portion 2a comprend une interface de coulissement (non représentée) qui coopère avec le rail 2b. Cette interface de coulissement est montée ou intégrée à la périphérie de la première portion 2a de la structure support 2 de sorte que, en étant positionné à l'intérieur du moyen de protection 5, la première portion 2a de la structure support 2 coopère avec le rail 2b du moyen de protection 5 pour déplacer l'interface d'accumulation 3 en translation selon un axe parallèle à l'axe du rail 2b. De façon préférée, le déplacement de l'interface d'accumulation 3 par la première portion 2a fait intervenir au moins deux interfaces de coulissement positionnées de façon symétrique autour de l'axe de l'interface d'accumulation 3, chacune des interfaces de coulissement coopérant avec un rail 2b respectif du moyen de protection 5.

Selon un autre exemple particulier de construction du dispositif de prélèvement 1 de l'invention, complémentaire des particularités précédemment détaillées, l'interface d'accumulation 3 éventuellement en association avec la structure support 2, est montée de façon réversible avec le moyen de protection 5. Cet assemblage réversible est réalisé de telle sorte que l'interface d'accumulation 3 est insérée à l'intérieur d'au moins une partie de la cage que forme le moyen de protection 5. L'interface d'accumulation 3 est retirée du moyen de protection 5 par une translation, par exemple de type télescopique, de l'interface d'accumulation 3 par rapport au moyen de protection 5 de sorte que l'interface d'accumulation 5 est retirée à une extrémité du moyen de protection 5. Selon une spécificité de cette particularité de construction, l'extrémité de l'interface d'accumulation 3 est associée à un moyen de raclage 8 arrangé pour frotter contre la face intérieure du moyen de protection 5 et récupérer des éléments déposés ou qui seraient détachés de l'interface d'accumulation 3. Selon un exemple non-limitatif de construction, le moyen de raclage 8 est réalisé sous la forme d'une pièce circulaire de forme complémentaire à la section intérieure du moyen de protection 5.

Selon un autre exemple particulier de construction du dispositif de prélèvement 1 de l'invention, complémentaire des particularités précédemment détaillées, le dispositif de prélèvement 1 comprend au moins un mécanisme d'essorage d'au moins une partie de l'interface d'accumulation 3. Ce mécanisme d'essorage est intégré au dispositif de prélèvement 1 de sorte que, lors du retrait de l'interface d'accumulation 3, celle-ci se trouve automatiquement essorée pour permettre la récupération des matériaux et éléments qui s'y sont déposés et accumulés.

Selon un exemple spécifique de construction, le mécanisme d'essorage comprend au moins une interface de pincement 9 de l'interface d'accumulation 3, cette interface de pincement 9 étant montée, directement ou indirectement, sur au moins l'un des éléments qui réalisent la structure support 2 du dispositif 1. L'interface de pincement 9 est configurée pour opérer un écrasement, au moins ponctuel, de l'interface d'accumulation 3. Selon un arrangement préféré, le pincement opéré par l'interface 9 est effectué dans un plan sensiblement perpendiculaire à l'axe de l'interface d'accumulation 3 ou de la structure support 2.

Selon un exemple préféré de construction de cette interface de pincement 9, celle-ci est configurée de sorte que l'interface d'accumulation 3 étant montée réversible par rapport à au moins une partie de la structure support 2, l'interface d'accumulation 3 est arrangée de façon à être retirée par coulissement selon un axe qui traverse l'interface de pincement 9. Aussi, le retrait de l'interface d'accumulation 3 de l'intérieur du moyen de protection 5 permet d'opérer conjointement un pincement de l'interface d'accumulation 3 progressif sur toute la longueur de l'interface d'accumulation 3 et donc un essorage de cette interface d'accumulation 3 de façon à en extraire les matériaux et éléments qui s'y sont déposés et accumulés.

Selon un exemple particulier de construction de cette interface de pincement 9, celle-ci comprend deux éléments axiaux 9a respectifs arrangés de façon sensiblement parallèle entre eux et configurés pour réaliser un écartement dont la distance est inférieure à la largeur d'au moins une portion de l'interface d'accumulation 3 arrangée pour être mobile par rapport aux surfaces de ces deux éléments axiaux 9a. Aussi, selon cet exemple de construction, le déplacement de l'interface d'accumulation 3 entre les deux éléments axiaux 9a de l'interface de pincement 9 conduit à une compression de l'interface d'accumulation 3 dans un plan perpendiculaire à l'axe de l'interface d'accumulation 3. Par cette compression par les deux éléments axiaux 9a, l'interface d'accumulation 3 se trouve ainsi essorer.

Selon une variante spécifique de construction de cette interface de pincement 9 réalisée à partir d'une paire d'éléments axiaux 9a, au moins un des éléments axiaux 9a est associé à un rouleau 9b monté mobile en rotation autour de l'élément axial 9a. Ce rouleau 9b réalise ainsi une surface de frottement destinée à appuyer contre la surface de l'interface d'accumulation 3. Avec la pression exercée par l'interface de pincement 9 contre l'interface d'accumulation 3, la surface du rouleau 9b réalise une surface de roulement qui facilite le déplacement de l'interface d'accumulation 3 entre les deux éléments axiaux 9a de l'interface de pincement 9. Selon une construction préférée de cette variante spécifique de construction, l'interface de pincement 9 comprend au moins un rouleau 9a monté pivotant autour de chacun des deux éléments axiaux 9a.

Selon une autre variante spécifique de construction, complémentaire de la variante spécifique précédemment détaillée, au moins un premier des éléments axiaux 9a est monté amovible par rapport au second des éléments axiaux 9a et/ou à au moins l'un des éléments qui réalisent la structure support 2 du dispositif 1 et notamment l'élément de la structure support 2 qui porte l'interface de pincement 9 du mécanisme d'essorage. Selon un exemple de cette variante spécifique de construction, au moins un des éléments axiaux 9a comprend un coude 9c à l'une de ses extrémités et au niveau duquel cet élément axial 9a est monté pivotant pour être écarté du second élément axial 9a. Le pivotement de l'élément axial 9a pour permettre son écartement est effectué autour d'un axe différent et non-parallèle par rapport à l'axe propre de l'élément axial 9a. Aussi, selon cet exemple de construction, cet élément axial 9a est fixé sur un élément de la structure support 2, d'une part, au niveau du coude 9c positionné à une première extrémité et qui réalise un point d'articulation 9d en rotation avec la structure support 2 et, d'autre part, au niveau d'un point de verrouillage 9e réversible positionné à une seconde extrémité de l'élément axial 9a.

Selon un autre exemple spécifique de construction, le mécanisme d'essorage intègre au moins une interface de pincement 9 qui comprend, au niveau d'au moins une portion de sa structure, un arrangement sensiblement conique 90 dont la largeur la plus rétrécie 91 est inférieure à la largeur d'au moins une portion de l'interface d'accumulation 3. Selon cet exemple spécifique de construction, le déplacement de l'interface d'accumulation 3 au travers de l'arrangement conique 90 du mécanisme d'essorage se traduit par une compression périphérique progressive et localisée de l'interface d'accumulation 3 de sorte que les matériaux et éléments qui s'y sont déposés et accumulés s'en trouvent extraits et sont en mesure d'être récupérés.

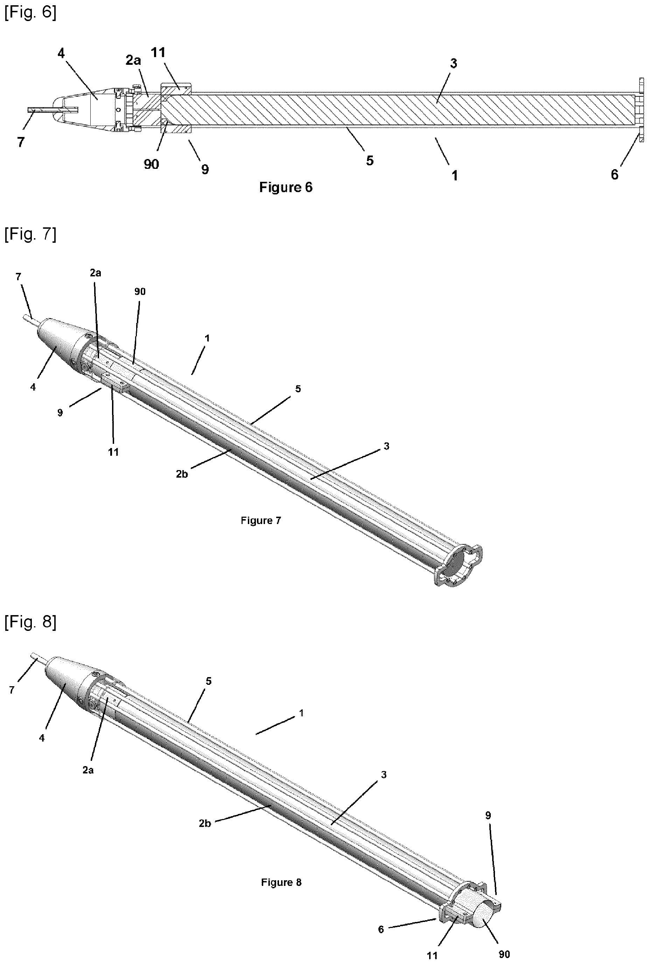

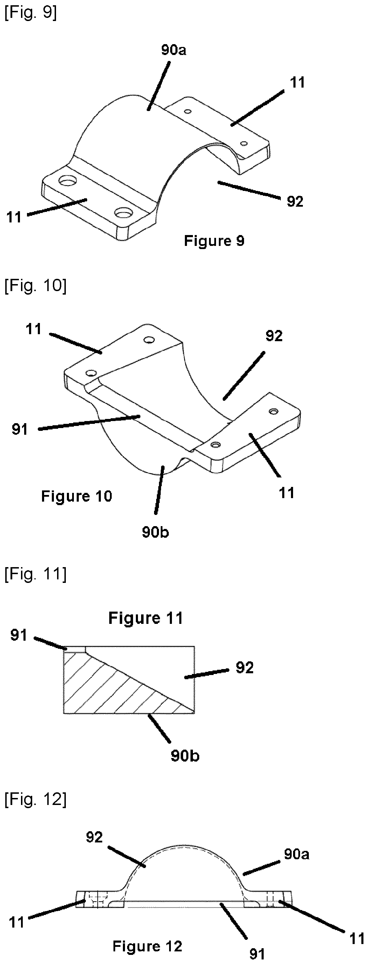

Selon un exemple de construction de cet arrangement conique 90 de l'interface de pincement 9, celui-ci est réalisé sous la forme de deux moitiés de cônes 90a, 90b sensiblement identiques et configurées pour être assemblées par positionnement en vis-à-vis de part et d'autre d'un plan de symétrie de l'arrangement conique 90. Selon un exemple particulier, le maintien assemblé des deux moitiés de cônes 90a, 90b est réalisé par l'intermédiaire de portions 11 positionnées latéralement, en périphérie de la surface extérieure de l'arrangement conique 90. Ces portions latérales 11 forment des surfaces de contact au niveau desquels des éléments de serrage, tels que des vis, sont susceptibles d'être positionnés. Selon un autre exemple particulier compatible de cette construction, l'arrangement conique 90 présente, de part et d'autre de ses extrémités, une ouverture large 92 et une ouverture réduite 91. Dans un plan en section perpendiculaire à l'axe de l'interface d'accumulation, l'ouverture large 92 présente une section de forme et de dimension sensiblement similaire à celle de l'interface d'accumulation 3. En revanche, l'ouverture réduite 91 est susceptible d'être réalisée selon toute forme en section arrangée pour comprimer l'interface d'accumulation 3 dans un plan perpendiculaire à son axe. Selon une construction illustrée par les figures 6, 9, 10, 11 et 12 et proposée à titre d'exemple, l'ouverture réduite 91 de l'arrangement conique 90 présente une forme aplatie dont la largeur correspond sensiblement à celle de l'ouverture large 92 mais dont la hauteur est suffisamment réduite pour pincer l'interface d'accumulation 3 qui traverse cet arrangement conique 90 et permettre son essorage. De façon préférée, cette ouverture réduite 91 est, comme l'ouverture large 92, centrée sur l'axe de l'interface d'accumulation 3.

Selon un autre exemple particulier de construction, le mécanisme d'essorage comprend également au moins un rail 2b positionné dans la longueur du moyen de protection 5, l'interface de pincement 9 étant montée mobile sur le rail 2b à l'intérieur du moyen de protection 5, en périphérie de l'interface d'accumulation 3, de sorte que le déplacement de l'interface de pincement 9 sur le rail 2b entraine un pincement de l'interface d'accumulation 3 logé dans le moyen de protection 5 le long d'au moins la partie de la longueur correspondant au déplacement. Aussi, selon cet exemple particulier de construction du mécanisme d'essorage de l'interface d'accumulation 3, l'interface d'accumulation 3 est essorée en demeurant fixe par rapport à la structure support 2 et notamment par rapport au moyen de protection 5. L'interface de pincement 9 est déplacée le long du rail 2b pour compresser l'interface d'accumulation 3 et essorer celle-ci progressivement de proche en proche le long de sa longueur. Ainsi, le déplacement de l'interface de pincement 9 le long de ce rail 2b réalise conjointement un déplacement du point de compression ou de la section de compression de l'interface d'accumulation 3 au niveau de sa longueur et l'essorage qui s'y rattache. Selon un arrangement préféré, de cet exemple particulier de construction, le mécanisme d'essorage comprend deux rails 2b portés par le moyen de protection 5 de la structure support 2 et positionnés de part et d'autre de l'interface d'accumulation 3. Cet arrangement préféré permet la réalisation d'un guidage optimisé du déplacement de l'interface de pincement 9 dans la longueur de l'interface d'accumulation 3.

Selon une particularité de construction, l'interface de pincement 9 comprend au moins une interface de coulissement qui coopère avec un rail 2b respectif du mécanisme d'essorage en étant mobile en translation par rapport au moyen de protection 5 de la structure support 2 et selon un axe parallèle à l'axe d'un rail 2b. De façon préférée, dans un plan en section perpendiculaire à l'axe du rail 2b, cette interface de coulissement est montée ou intégrée à une portion de la périphérie de l'interface de pincement 9, de façon à coopérer avec un rail 2b respectif du moyen de protection 5. Idéalement, le déplacement de l'interface de pincement 9 fait intervenir au moins deux interfaces de coulissement positionnées de façon symétrique autour de l'axe de l'interface d'accumulation 3. Selon un exemple de construction illustré sur les figures 6, 7, 8, 9 et 10, l'interface de pincement 9 réalisée par un arrangement conique 90 comprend des portions latérales 11 qui participent au maintien de la réunion des deux moitiés de cônes 90a, 90b, forment également des interfaces de coulissement par coopération avec un rail 2b respectif du moyen de protection 5. Les interfaces de coulissement sont ici intégrées à l'interface de pincement 9.

A titre d'exemple complémentaire également représenté sur les figures 6, 7 et 8, un rail 2b intégré à la structure du moyen de protection 5 est réalisé sous la forme d'une paire de tiges parallèles. L'élément de coulissement 11 réalisé par les portions latérales 11 de l'arrangement conique 90 est ainsi maintenu positionné entre ces deux tiges parallèles, de sorte que le déplacement de cet élément de coulissement soit guidé en translation le long d'un axe parallèle aux tiges qui forment le rail 2b. Conjointement, au déplacement de cet élément de coulissement, l'interface de pincement 9 se trouve également translatée selon un axe parallèle à l'axe du rail 2b, c'est-à-dire le long de l'axe de l'interface d'accumulation 3.

Bien entendu, l'invention n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention.

## Revendications

1. Dispositif de prélèvement (1) en milieu aqueux, **caractérisé en ce que** le dispositif (1) comprend :
- une structure support (2) rectiligne,
- une interface d'accumulation (3) montée sur au moins une partie de la structure support (2),
- un moyen de flottaison (4) monté à une extrémité de la structure support (2) rectiligne.

2. Dispositif de prélèvement (1) selon la revendication 1, **caractérisé en ce que** la structure support (2) comprend également un moyen de protection (5) qui entoure au moins une partie de l'interface d'accumulation (3).

3. Dispositif de prélèvement (1) selon une des revendications précédentes, **caractérisé en ce que** l'interface d'accumulation (3) comprend au moins un matériau de type spongieux.

4. Dispositif de prélèvement (1) selon une des revendications précédentes, **caractérisé en ce que** le dispositif (1) comprend au moins un mécanisme d'essorage d'au moins une partie de l'interface d'accumulation (3).

5. Dispositif de prélèvement (1) selon la revendication 4, **caractérisé en ce que** le mécanisme d'essorage comprend au moins une interface de pincement (9) de l'interface d'accumulation (3), cette interface de pincement étant montée, directement ou indirectement, sur au moins une partie de la structure support 2.

6. Dispositif de prélèvement (1) selon la revendication 5, **caractérisé en ce que** le mécanisme d'essorage comprend également au moins un rail (2b) positionné dans la longueur du moyen de protection (5), l'interface de pincement (9) étant montée mobile sur le rail (2b) à l'intérieur du moyen de protection (5), en périphérie de l'interface d'accumulation (3), de sorte que le déplacement de l'interface de pincement (9) sur le rail (2b) entraine un pincement de l'interface d'accumulation (3) logé dans le moyen de protection (5) le long d'au moins la partie de la longueur correspondant au déplacement.

7. Dispositif de prélèvement (1) selon la revendication 5, **caractérisé en ce que**, l'interface d'accumulation (3) étant montée réversible par rapport à au moins une partie de la structure support (2), l'interface d'accumulation (3) est arrangée de façon à être retirée par coulissement selon un axe qui traverse l'interface de pincement (9).

8. Dispositif de prélèvement (1) selon une des revendications 5 à 7, **caractérisé en ce que** l'interface de pincement (9) comprend deux éléments axiaux (9a) respectifs arrangés de façon sensiblement parallèle entre eux et configurés pour réaliser un écartement dont la distance est inférieure à la largeur d'au moins une portion de l'interface d'accumulation (3), l'interface d'accumulation étant arrangée pour être mobile par rapport aux surfaces de ces deux éléments axiaux (9a).

9. Dispositif de prélèvement (1) selon la revendication 8, **caractérisé en ce que** au moins un premier des éléments axiaux (9a) est monté amovible par rapport au second des éléments axiaux (9a) et/ou à au moins l'un des éléments qui réalisent la structure support (2) du dispositif (1).

10. Dispositif de prélèvement (1) selon une des revendications 5 à 7, **caractérisé en ce que** le mécanisme d'essorage intègre au moins une interface de pincement (9) qui comprend, au niveau d'au moins une portion de sa structure, un arrangement sensiblement conique (90) dont la largeur la plus rétrécie est inférieure à la largeur d'au moins une portion de l'interface d'accumulation (3).

11. Dispositif de prélèvement (1) selon au moins la revendication 6, **caractérisé en ce que** l'interface de pincement (9) comprend au moins une interface de coulissement qui coopère avec un rail (2b) respectif du mécanisme d'essorage en étant mobile en translation par rapport au moyen de protection (5) de la structure support (2) et selon un axe parallèle à l'axe d'un rail (2b).
